# EUROPEAN PATENT SPECIFICATION

(11) **EP 3 220 983 B1**
(45) Date of publication and mention of the grant of the patent: **19.12.2018**
(21) Application number: 15804967.6
(22) Date of filing: 30.10.2015
(51) Int. Cl.: A61M 5/32, A61M 25/06

(54) **DEVICE FOR ENTERING A BLOOD VESSEL USING A WINGED NEEDLE WITH POST-USE PROTECTION**
VORRICHTUNG ZUM EINTRITT IN EIN BLUTGEFÄSS MITHILFE EINER FLÜGELNADEL MIT SCHUTZ NACH DER VERWENDUNG
DISPOSITIF DESTINÉ À ENTRER DANS UN VAISSEAU SANGUIN À L'AIDE D'UNE AIGUILLE À AILETTE AVEC UNE PROTECTION POST-UTILISATION

(30) Priority: 20.11.2014 IT PD20140313
(43) Date of publication of application: 27.09.2017
(73) Proprietor: Vacutest Kima S.r.l., 35020 Arzergrande, Padova (IT)
(72) Inventor: CHIARIN, Renzo, 35020 Arzergrande, Padova (IT); CHIARIN, Ulisse, 35020 Arzergrande, Padova (IT)
(74) Representative: Zanettin, Gianluigi
(86) International application number: PCT/IB2015/058380
(87) International publication number: WO 2016/079623

(56) References cited:
- US-A- 5 120 320
- US-A1- 2004 153 039
- US-A1- 2013 116 598
- US-A1- 2013 184 657

## Description

### FIELD OF APPLICATION

This invention relates to a device for entering a blood vessel using a winged needle with post-use protection.

### STATE OF THE ART

"Winged needle" means a needle assembly or body equipped with a tubular support element from which two wings, which act as a grip for the handling of the whole needle body, extend laterally and the distal portion of a needle for entering a blood vessel extends longitudinally. The proximal portion of the needle is inserted in the tubular body and is fluidically connected to a tube by means of the same tubular body.

Devices are known for entering a blood vessel using a winged needle, equipped with a protection body that is applied on the distal portion of the needle after use so as to reduce the risk of accidental needle sticks for the operators.

In particular, devices are known for entering a blood vessel using a winged needle in which the protection body is slidably connected to the body of the winged needle. An example of such a device is disclosed in US2013/0116598A1.

More in detail, the device has a hollow protection body that defines an inner chamber inside which the needle body is inserted so as to slide, with the two wings that protrude from the protection body through two lateral slide slits that extend opposite each other between said two ends. The needle body is slidingly movable along the inner chamber from a bare needle position, in which it is positioned in proximity of the front end of the protection body with the distal portion of the needle coming out through an exit opening made tehrin, to a protected needle position, in which it is positioned in proximity of the rear end of the protection body with the needle completely retracted within the protection body itself.

In proximity of the rear end of the protection body each slit has a first section with a widened lumen which defines a seat inside which seat the corresponding wing of the needle body is engaged when the latter is brought into the protected needle position. The seat is shaped so as to guarantee an irreversible locking of the needle body.

In proximity of the front end of the protection body each slit has a second section with a widened lumen which defines a holding seat for the needle body when the latter is in a bare needle position.

Generally, entry devices with winged needle equipped with post-use protection body as described above, are supplied ready for use, with the needle arranged in uncovered position. However, the needle is protected by a protective cap which must be removed before use.

Operationally, for ease of assembling, it is preferable to associate the winged needle to the protection body already equipped with the removable protective cap. The hollow, post-use protection body is formed from two separate half-shells, provided with interconnection elements. In particular, the two half-shells are stably connected to each other at the front end, for example via one or more connecting portions that develop on both sides of the exit opening. Assembling is done by arranging the two half-shells open and then inserting the needle with the relative cap through the exit opening. Once the cap is positioned completely outside of the protection body, the two half-shells are pushed together and permanently interconnected to one another so as to enclose the tubular support element inside them.

The size of the exit opening must be dimensioned so as to allow the passage of the whole cap. In particular, it is dimensioned so that it allows the portion of the cap in which the mouth is formed to pass through. In fact, this portion is generally larger than the rest of the cap, since it is dimensioned to receive part of the tubular body so as to be coupled stably to it.

Generally, the exit opening is formed on one of the two half-shells, in correspondence of the front end of the protection body. To facilitate the passage of the needle with the relative cap, the exit opening is enlarged, extending it in the direction also longitudinal to the other half-shell. In this way, however, the opening is no longer limited to the front end of the protection body, but also involves it in the longitudinal direction. The chamber inside which the needle body is retracted is thus partially accessible, and precisely near the end portion of the needle, i.e., the most dangerous part that you actually want to shield.

Therefore, the need is felt to simplify the assembling of a winged device, making possible the use of even protective caps of large size, without, however, making accessible in the longitudinal direction the inner chamber in which the end portion of the needle is housed in the post-use, needle-protected position.

### PRESENTATION OF THE INVENTION

Therefore, the purpose of this invention is to eliminate or at least mitigate the drawbacks of the prior art cited above, by making available a device for entering a blood vessel using a winged needle with the post-use protection that it is more easily assembled even with needle already protected by a cap without making longitudinally accessible the inner chamber in which the end portion of the needle is housed in the post-use, needle-protected position.

A further purpose of this invention is to make available a device for entering a blood vessel using a winged device with post-use protection that is easily and readily usable.

A further purpose of this invention is to make available a device for entering a blood vessel using a winged device with post-use protection that is easy and economical to produce.

### BRIEF DESCRIPTION OF THE DRAWINGS

The technical characteristics of the invention, according to the above-mentioned purposes, can be clearly understood from the claims listed below and its advantages will become more apparent from the detailed description that follows, made with reference to the attached drawings, which show one or more purely exemplary and non-limiting embodiments wherein:
- Figure 1 is a perspective view of a device for entering a blood vessel using a winged needle with post-use protection according to a preferred embodiment of the invention, illustrated with the needle body in a bare needle position and provided with a removable protective cap;
- Figure 2 is a longitudinal cross-section view of the device of Figure 1 according to the line II-II indicated therein;
- Figure 3 is an enlarged view of a detail of Figure 2 relative to the exit opening of the needle and highlighted in the circle III shown therein;
- Figure 4 is a perspective view of the device of Figure 1 illustrated in an initial assembling step of the needle body (already provided with removable cap) on the hollow post-use protection body;
- Figure 5 is a partially transparent side view of the device illustrated in Figure 4;
- Figure 6 is a perspective view of the device of Figure 1 illustrated in an intermediate assembling step in which the needle body (already provided with removable cap) is associated to the hollow post-use protection body;
- Figure 7 is a partially transparent side view of the device illustrated in Figure 4;
- Figure 8 is a perspective view of the device of Figure 1 in a final assembling step in which the needle body provided with removable cap is already associated to the hollow post-use protection body;
- Figure 9 is a longitudinal cross-section view of the device of Figure 8 according to the line II-II indicated therein;
- Figure 10 is an enlarged view of a detail of Figure 9 relative to the exit opening of the needle and highlighted in the circle X shown therein;
- Figure 11 is a perspective view of an entry device of Figure 1, illustrated in assembled condition post-use, with the needle body retracted in the needle-protected position; and
- Figure 12 is a longitudinal cross-section view of the device of Figure 11 according to the line XII-XII indicated therein.

### DETAILED DESCRIPTION

With reference to the accompanying drawings, the reference number 1 indicates in its entirety a device for entering a blood vessel using a winged needle with post-use protection.

Here and in the following description and claims, reference will be made to the device 1 in condition of use. In this sense, any references to a lower or higher or front or rear position or a horizontal or vertical direction must be understood in this sense.

According to a general embodiment of the invention, the device 1 for entering a blood vessel using a winged needle with post-use protection, comprises:
- a winged needle body 10 provided with a tubular support element 11 from which two wings 12 extend laterally in opposite directions, and from which the distal portion 14 of a needle 13 extends longitudinally for entering a blood vessel;
- a hollow protection body 20, which extends between a front end 21 and a rear end 22, defining an inner chamber 23 inside which the needle body is inserted so as to slide, with the two wings which protrude from the protection body through two lateral slide slits 24 which extend opposite each other between said two ends 21,22.

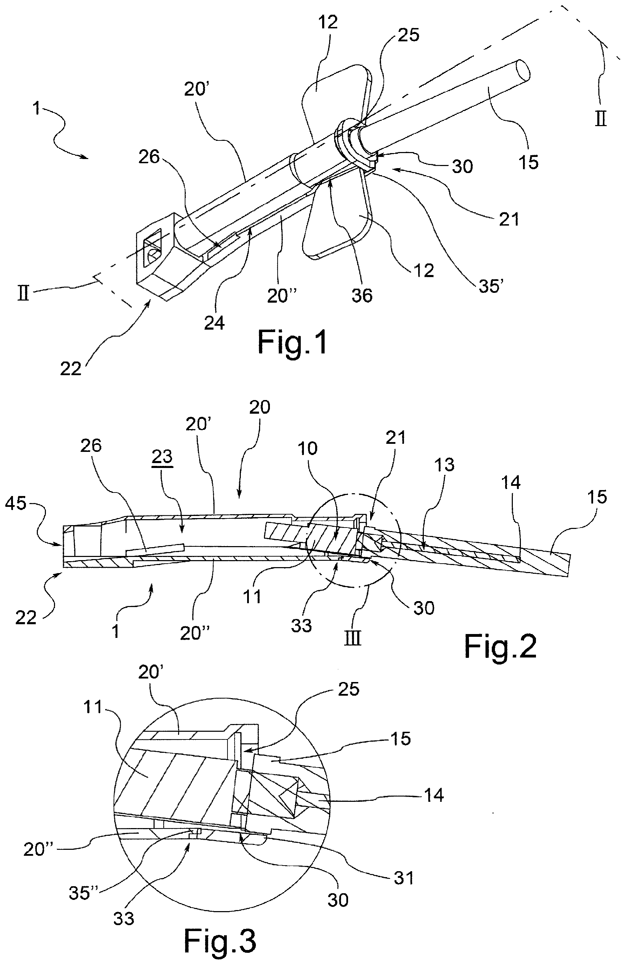

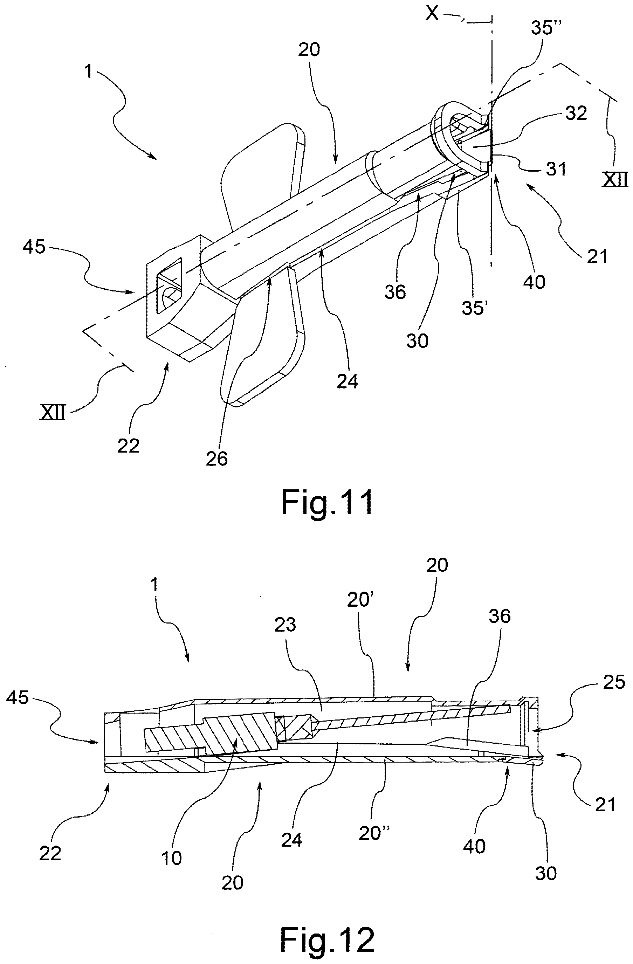

Operatively, the needle body 10 is slidingly movable along the inner chamber 23 from a bare needle position, in which it is positioned in proximity of the front end 21 of the protection body 20 with the distal portion 14 of the needle coming out through an exit opening 25 formed on it and is protected - before use - by a removable cap 15 (see Figures 1 and 2), to a protected needle position, in which the needle body 10 - freed of the protection cap 15 - is positioned in proximity of the rear end 22 of the protection body with the needle completely retracted within the protection body itself (see Figures 11 and 12).

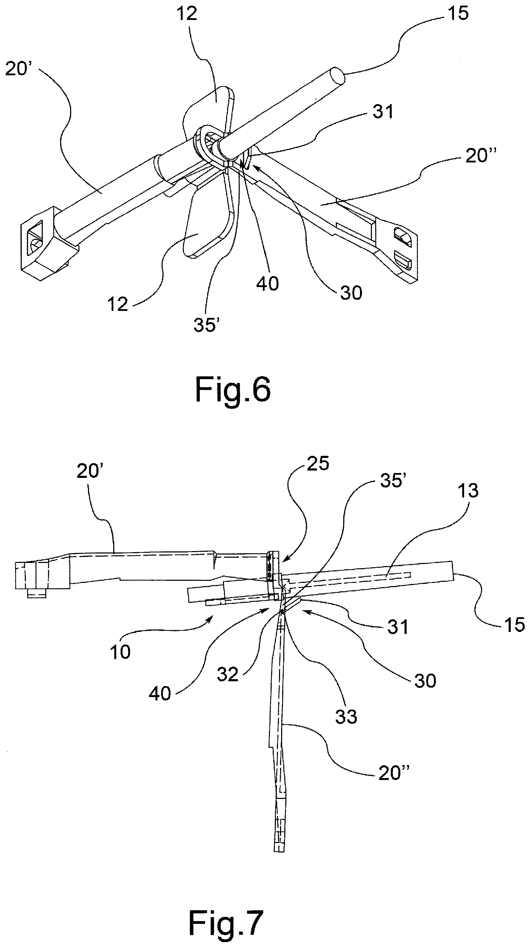

As illustrated in Figure 6, in a portion of the hollow protection body 20 which delimits the inner chamber 23, in the vicinity of the outlet opening 25 an additional opening 40 is formed, which additional opening connects the inner chamber 23 with the outside and extends in the longitudinal direction towards the front end 21 to the exit opening 25.

The protection body 20 is divided by the two slits 24 in two half-shells 20' and 20".

The two half-shells 20' and 20" are connected to each other at least at the front end 21 of the protection body itself in such a way that the inner chamber 23 of the hollow protection body 20 can be opened by moving apart the two half-shells 20' and 20" from each other in correspondence of the rear portion 22 with a rotation around a rotation axis X passing near the exit opening 25.

The division into two half-shells is functional to the assembling of the device 1, as will be clarified in the description below.

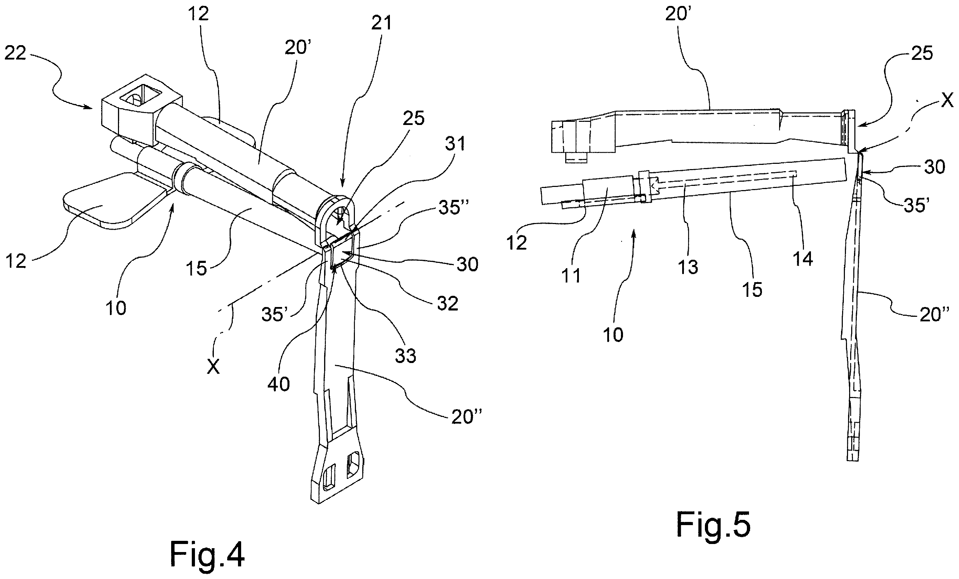

According to an essential aspect of this invention, as illustrated in particular in Figure 4, the aforesaid additional opening 40 is at least partially closed by a tongue 30, which extends projecting from the hollow protection body 20 in the longitudinal direction towards the front end 21.

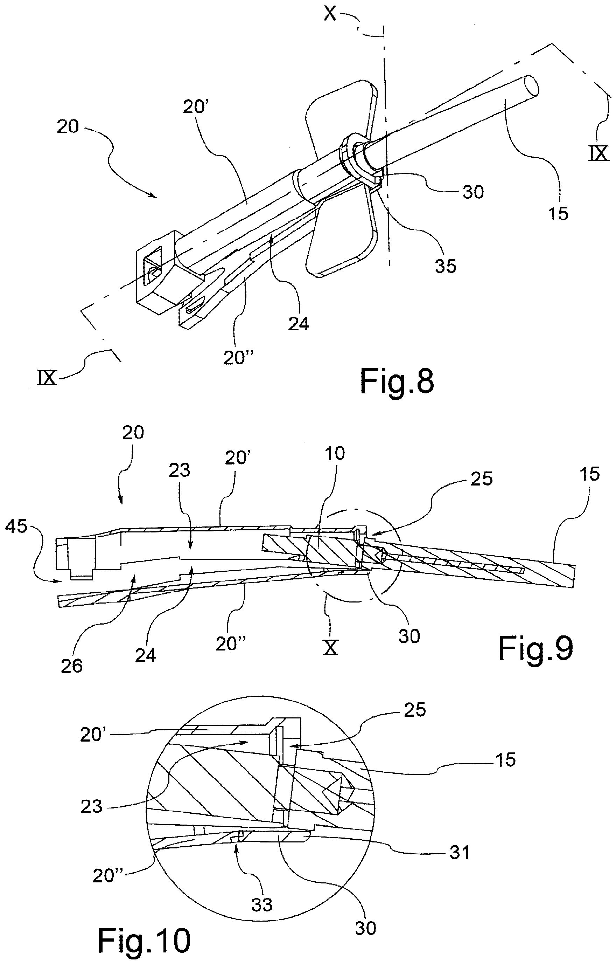

As illustrated in the sequence of Figures 4, 6, 8 and 9, the aforesaid tongue 30 is movable between a rest position, in which the tongue 30 at least partially closes the additional opening 40 reducing the open lumen of additional opening 40 (see Figures 8 and 11), and an operating position, in which the tongue 30 is divaricated from the hollow protection body so as to leave the additional opening 40 open and to widen the exit opening 25 (see Figures 6 and 7).

Operationally, the movement of the tongue 30 from the rest position to the operative position can be exploited during assembling of the device 1, associating to the hollow protection body the winged needle already equipped with removable protection cap.

More in detail, the assembling is done by preparing the two open half-shells open (condition of internal chamber open, Fig. 4) and then inserting the needle with the related cap through the exit opening 25 (Fig. 6). Once the cap is positioned completely outside of the protection body (Fig. 6), the two half-shells are pushed together again (condition of inner chamber closed, Fig. 8) and permanently interconnected to one another so as to enclose the tubular support element 10 inside them (Fig. 1) .

The insertion of the needle with its relative cap through the exit opening 25 is facilitated by the presence of the additional opening 40, which widens the lumen of the exit opening 25 at the time of the passage of the cap 15. It is thus possible to provide the use of even large caps. In dimensioning the device 1, and in particular the hollow protection body 20, one can therefore exploit the additional passage lumen offered by the additional opening 40.

Thanks to the invention, and in particular thanks to the shielding offered by the projecting tongue 30, the additional opening 40 may extend in the longitudinal direction without any limit. In fact, the presence of the tongue 30 eliminates the risk of making accessible from outside the inner chamber 23 of the protection body when the needle is in the needle-protected position.

It is therefore possible to simplify the assembling of the device by providing an additional opening even very extended, without reducing in any way the protection offered by the hollow protection body.

A further advantage associated with this invention lies in the fact that the same hollow protection body can be used for needle bodies of different sizes or at least provided with removable protection caps of different sizes. A hollow protection body is made with an additional opening dimensioned for the larger needle body or cap, to then also be used with smaller needle bodies or caps. It is therefore possible to standardise the size of the protection body, avoiding the diversification of production as a function of the size of the protection caps or needle bodies.

Preferably, as illustrated in the accompanying drawings, in the rest position the projecting tongue 30 closes the aforesaid additional opening 40 for its entire longitudinal extension, preventing accidental access to the inner chamber from the outside in correspondence of said additional opening 40.

In particular, at least in the direction of transversal development, the closure of the additional opening 40 by the projecting tongue 30 must not necessarily be flush, i.e., the tongue need not necessarily be in contact with the longitudinal edges of the additional opening to occupy all the lumen. For example, for construction reasons, it is possible to provide that, between the tongue and the edges of the opening, there is play of reduced lumen, for example a few tenths of a millimetre. This lumen is sufficient to ensure free movement of the tongue, avoiding interference with the longitudinal edges, without, however, creating the risk of an accidental access to the chamber or the exit of the needle.

Preferably, the projecting tongue 30 extends longitudinally up to the exit opening 25. In particular, the free end 31 of the tongue 30 can contribute to defining the contour of the exit opening 25. It is preferable that the tongue 30 extends for the entire longitudinal development of the additional opening 40 in such a way that there is no longitudinal portion of the additional opening 40 uncovered.

According to non-preferred alternative embodiments not illustrated in the accompanying drawings, the tongue 30 can have a longitudinal extension less than that of the additional opening 40. In any case, the advantage associated to this invention continues to be present as it is possible to make the additional opening of longitudinal dimensions greater with respect to the case where the aforesaid projecting tongue is not present.

Advantageously, the aforesaid projecting tongue is made in one piece with the hollow protection body 20.

Preferably, the projecting tongue 30 is made as an elastically flexible element, suitable to move elastically from the aforesaid rest position to the aforesaid operative position when stressed in this direction (by the removable cap 15 pushed from the inside outwards against the tongue 30) and to automatically return to the rest position in the absence of stress, when the needle with relative cap has completely passed through the exit opening and the additional opening and no longer interferes with the tongue 30. In this way it is possible to ensure that the tongue 30 returns to the rest position, ensuring the protection of the inner chamber in which the needle is placed after use.

According to the preferred embodiment illustrated in the accompanying drawings, and in particular in Figure 4, the projecting tongue 30 has an incision 33 made in correspondence of a connection base 32 to the protection body 20. This incision 33 defines a preferential bending line of the tongue 30.

Preferably, the incision 33 is substantially transversal to the longitudinal development of the hollow protection body 20 and the tongue 30 itself.

In particular, as illustrated in Figures 6 and 7, the incision 33 is made so as to facilitate the bending of the tongue 30 towards the outside of the inner chamber 23.

According to the preferred embodiment illustrated in the accompanying drawings, and in particular in Figures 4 and 6, the additional opening 40 is delimited longitudinally by two appendixes 35' and 35" of the hollow protection body 20, between which the projecting tongue 30 is arranged when it is in the rest position. In particular, these appendixes define the longitudinal edges of the additional opening 40.

Advantageously, in the open chamber configuration (see Figure 5), the two half-shells 20' and 20" can be arranged relative to one another in such a way that the tongue 30 in the rest position is substantially coplanar or parallel to a plane on which the exit opening 25 lies. This can facilitate insertion of the needle with the relative cap 15 in the exit opening 25 and the additional opening 40.

Preferably, as illustrated in the accompanying drawings, the exit opening 25 is made on a first half-shell 20' and the additional opening 40 is made on the second half-shell 20".

In particular, the two half-shells 20' and 20" are connected together at least at the front end 21 of the protection body 20 at the front ends of aforesaid two appendixes 35' and 35". Advantageously, these two appendixes 35' and 35" are formed in one piece with the two half-shells. The rotation axis X passes through the front ends of the aforesaid two appendixes 35' and 35".

In particular, at the rear end 22, the two half-shells are completely separate and are connected by means of interlocking elements.

Preferably, the protection body is made of plastic with injection-moulding techniques.

In particular, the protection body 20 has, in correspondence of the rear end 22, a through-opening 45 for a tube (not illustrated in the figures) connected to the tubular body of the winged needle in a position longitudinally opposite the needle 13.

According to a preferred embodiment of the invention illustrated in the accompanying drawings, in proximity of the rear end of the protection body, each slit 24 has a first section with a widened lumen 26 which defines a blocking seat inside which seat the corresponding wing 12 of the needle body is engaged when the latter is brought into the protected needle position. The seat 26 is shaped so as to guarantee an irreversible locking of the needle body

In proximity of the front end 21 of the protection body 20, each slit 24 has a second section with a widened lumen 36 which defines a holding seat for the needle body 10 when the latter is in a bare needle position. In particular, the holding seat has a sufficient lumen to accommodate the corresponding wing 12 of the needle body and is shaped so as to ensure a reversible positioning of the needle body inside it. In particular, the edges of the slit portion that defines the holding seat form a guide zone to facilitate the exit of the corresponding wing from the seat 36 and thus its entry into the section of slit that leads to the blocking seat 26.

Advantageously, in each slit 24, at least for an intermediate section positioned between the blocking seat 26 and the front end 21 of the protection body 20 (and in particular between the blocking seat and the holding seat) the two opposite edges of the same slit 24 are in contact with each other or at least very close together. According to a preferred embodiment, this intermediate portion extends between the blocking seat 26 and the holding seat 36. Preferably, this intermediate section borders on the blocking seat 26.

This invention also covers a hollow, post-use protection body for devices for entering a blood vessel using a winged needle, i.e., for devices provided with a needle body provided with a tubular support element from which two wings extend laterally in opposite directions and the distal portion of a needle for entering a blood vessel extends longitudinally.

This protection body has the same characteristics of the hollow protection body 20 previously described in relation to the device 1. For simplicity of exposition, the description of this protection body will not be repeated here, being understood that what was said previously is valid.

The invention allows obtaining several advantages, some of which have already been highlighted previously.

The device for entering a blood vessel using a winged needle with the post-use protection according to the invention can be assembled more easily even with needle already protected by cap without, however, reducing the level of protection and safety offered by the device. In fact, the additional opening can be made with the desired longitudinal extension while maintaining, when the device is assembled, an adequate shielding of the inner chamber defined by the post-use protection body.

The device for entering a blood vessel using a winged needle with post-use protection according to the invention is also simple and immediate to use, since not specific additional manoeuvres are required with respect to conventional systems.

The device for entering a blood vessel using a winged needle with post-use protection according to the invention is also easy and economical to produce, since it can be made of plastic (with the exception of the needle, preferably of steel) with injection-moulding techniques.

Therefore, the invention thus conceived achieves the predefined purposes.

Obviously, it may even assume, in its practical embodiment, forms and configurations different from that illustrated above without, for this reason, departing from the present scope of protection.

Moreover, all the details may be replaced by technically equivalent elements and the dimensions, forms and materials used may be any according to the needs.

## Claims

1. Device for entering a blood vessel using a winged needle with post-use protection, comprising:
- a winged needle body (10) provided with a tubular support element (11) from which two wings (12) extend laterally in opposite directions, and from which the distal portion (14) of a needle (13) extends longitudinally for entering a blood vessel;
- a hollow protection body (20), which extends in the longitudinal direction between a front end (21) and a rear end (22), defining an inner chamber (23) inside which the needle body is inserted so as to slide, with the two wings which protrude from the protection body through two lateral slide slits (24) which extend opposite each other between said two ends (21, 22),
the needle body (10) being slidingly movable along the inner chamber (23) from a bare needle position, in which it is positioned in proximity of the front end (21) of the protection body (20) with the distal portion (14) of the needle coming out through an exit opening (25) made therein, to a protected needle position, in which it is positioned in proximity of the rear end (22) of the protection body with the needle completely retracted within said protection body, in a portion of the hollow protection body which delimits the inner chamber (23), in proximity of the exit opening (25) an additional opening (40) being provided, which additional opening extends in the longitudinal direction towards the front end (21) as far as the exit opening (25), the two slits (24) dividing the protection body (20) into two half-shells (20', 20"), the latter being connected to each other at least at the front end (21) of said protection body in such a way that the inner chamber (23) of the hollow protection body (20) can be opened by moving apart the two half-shells (20', 20") at the rear portion (22) with a through rotation around a rotation axis (X) passing in proximity of the exit opening (25),
**characterised in that** said additional opening (4'0) is at least partially closed by a tongue (30), which extends so as to project from the hollow protection body in the longitudinal direction towards the front end (21), said tongue (30) being movable between a rest position, in which the tongue (30) at least partially closes the additional opening (40) reducing the open lumen of said additional opening (40), and an operating position, in which the tongue (30) is divaricated from the hollow protection body so as to leave the additional opening (40) open and to widen the exit opening (25).

2. Device according to claim 1, wherein in the rest position the projecting tongue (30) closes said additional opening (40) all along its longitudinal extension, the free end (31) of said tongue (30) concurring to define the contour of the exit opening (25) .

3. Device according to claim 1 or 2, wherein the tongue (30) is made as an elastically flexible element, suitable to elastically shift from said rest position to said operating position when stressed in such direction and to automatically return to the rest position in the absence of stresses.

4. Device according to claim 3, wherein the projecting tongue (30) has, at a connection base (32) to the protection body (20), an incision (33) which defines a preferential bending line of the tongue (30), preferably said incision (33) being substantially transversal to the longitudinal extension of the hollow protection body (20) and of said tongue (30).

5. Device according to claim 4, wherein the incision (33) is made so as to facilitate the bending of the tongue (30) towards the outside of the inner chamber (23) .

6. Device according to one or more of the preceding claims, wherein the additional opening (40) is defined longitudinally by two appendixes (35', 35") of the hollow protection body (20) between which the projecting tongue (30) is positioned when in the rest position.

7. Device according to one or more of the previous claims, wherein in the open chamber configuration the two half-shells (20', 20") can be arranged relative to one another in such a way that the tongue (30) in the rest position is substantially coplanar or parallel to a plane on which the exit opening lies (25).

8. Device according to one or more of the previous claims, wherein the exit opening (25) is made on a first half-shell (20') and the additional opening (40) is made on the second half-shell (20"), the two half-shells (20', 20") being connected together at least at the front end (21) of the protection body (20) at the front ends of said two appendixes (35', 35").

9. Device according to one or more of the previous claims, wherein in proximity of the rear end (22) of the protection body each slit (24) has a first section with a widened lumen (26) which defines a blocking seat inside which seat the corresponding wing (12) of the needle body engages when it is brought into the protected needle position and wherein in proximity of the front end (21), of the protection body (20) each slit (24) has a second section with a widened lumen (26") which defines a holding seat (37) for the needle body when the latter is in a bare needle position.

10. Hollow, post-use, protection body (20) of the device according to claim 1.

## Patentansprüche

1. Vorrichtung zum Eintreten in ein Blutgefäß unter Verwendung einer geflügelten Nadel mit Nachverwendungsschutz, umfassend:
einen geflügelten Nadelkörper (10), der mit einem röhrenförmigen Stützelement (11) versehen ist, von dem sich zwei Flügel (12) seitlich in entgegengesetzten Richtungen erstrecken, und von dem sich der distale Abschnitt (14) einer Nadel (13) in Längsrichtung erstreckt, um in ein Blutgefäß einzutreten;
einen hohlen Schutzkörper (20), der sich in der Längsrichtung zwischen einem Vorderende (21) und einem Hinterende (22) erstreckt, wobei er eine Innenkammer (23) definiert, in die der Nadelkörper eingeführt wird, sodass er mit den zwei Flügeln, die von dem Schutzkörper hervorspringen, durch zwei seitlichen Gleitschlitze (24), die sich entgegengesetzt voneinander zwischen den zwei Enden (21, 22) erstrecken, gleitet, wobei der Nadelkörper (10) gleitend beweglich entlang der Innenkammer (23) von einer blanken Nadelposition ist, in der er in der Nähe des Vorderendes (21) des Schutzkörpers (20) positioniert ist, wobei der distale Abschnitt (14) der Nadel durch eine Austrittsöffnung (25), die darin vorgenommen ist, zu einer geschützten Nadelposition herauskommt, in der er in der Nähe des Hinterendes (22) des Schutzkörpers positioniert ist, wobei die Nadel vollständig innerhalb des Schutzkörpers eingezogen ist, in einem Abschnitt des hohlen Schutzkörpers, der die Innenkammer (23) begrenzt, wobei in der Nähe der Austrittsöffnung (25) eine zusätzliche Öffnung (40) vorgesehen ist, wobei sich die zusätzliche Öffnung in der Längsrichtung hin zu dem Vorderende (21) bis zu der Austrittsöffnung (25) erstreckt, wobei die zwei Schlitze (24) den Schutzkörper (20) in zwei Halbschalen (20', 20") teilen, wobei die letzteren miteinander mindestens an dem Vorderende (21) des Schutzkörpers derart verbunden sind, dass die Innenkammer (23) des hohlen Schutzkörpers (20) geöffnet werden kann, indem die zwei Halbschalen (20', 20") an dem Hinterabschnitt (22) mit einer Durchdrehung um eine Drehachse (X), die in der Nähe der Austrittsöffnung (25) verläuft, voneinander weg bewegt werden,
**dadurch gekennzeichnet, dass** die zusätzliche Öffnung (40) mindestens teilweise von einer Zunge (30) geschlossen ist, die sich so erstreckt, dass sie von dem hohlen Schutzkörper in der Längsrichtung hin zu dem Vorderende (21) hervorsteht, wobei die Zunge (30) zwischen einer Ruheposition, in der die Zunge (30) mindestens teilweise die zusätzliche Öffnung (40) schließt, wodurch das offene Lumen der zusätzlichen Öffnung (40) vermindert wird, und einer Betriebsposition, in der die Zunge (30) von dem hohlen Schutzkörper so gespreizt ist, dass sie die zusätzliche Öffnung (40) offen lässt und die Austrittsöffnung (25) weitet, beweglich ist.

2. Vorrichtung nach Anspruch 1, wobei in der Ruheposition die hervorstehende Zunge (30) die zusätzliche Öffnung (40) ganz entlang ihrer Längserstreckung schließt, wobei das freie Ende (31) der Zunge (30) übereinstimmt, um die Kontur der Austrittsöffnung (25) zu definieren.

3. Vorrichtung nach Anspruch 1 oder 2, wobei die Zunge (30) als ein elastisch flexibles Element hergestellt ist, das geeignet ist, sich von der Ruheposition zu der Betriebsposition elastisch zu verschieben, wenn es in einer solchen Richtung belastet wird, und in der Abwesenheit von Belastungen automatisch zu der Ruheposition zurückzukehren.

4. Vorrichtung nach Anspruch 3, wobei die hervorstehende Zunge (30) an einer Verbindungsbasis (32) zu dem Schutzkörper (20) eine Inzision (33) aufweist, die eine bevorzugte Biegelinie der Zunge (30) definiert, wobei bevorzugt die Inzision (33) im Wesentlichen quer zu der Längserstreckung des hohlen Schutzkörpers (20) und der Zunge (30) ist.

5. Vorrichtung nach Anspruch 4, wobei die Inzision (33) so vorgenommen ist, dass sie die Biegung der Zunge (30) hin zu der Außenseite der Innenkammer (23) erleichtert.

6. Vorrichtung nach einem oder mehreren der vorhergehenden Ansprüche, wobei die zusätzliche Öffnung (40) in Längsrichtung von zwei Fortsätzen (35', 35") des hohlen Schutzkörpers (20) definiert ist, zwischen denen die hervorstehende Zunge (30) positioniert ist, wenn sie sich in der Ruheposition befindet.

7. Vorrichtung nach einem oder mehreren der vorherigen Ansprüche, wobei die zwei Halbschalen (20', 20") in der offenen Kammerausgestaltung im Verhältnis zueinander derart angebracht werden können, dass die Zunge (30) in der Ruheposition im Wesentlichen koplanar oder parallel zu einer Ebene ist, auf der die Austrittsöffnung liegt (25).

8. Vorrichtung nach einem oder mehreren der vorhergehenden Ansprüche, wobei die Austrittsöffnung (25) auf einer ersten Halbschale (20') vorgenommen ist und die zusätzliche Öffnung (40) auf der zweiten Halbschale (20") vorgenommen ist, wobei die zwei Halbschalen (20', 20") miteinander mindestens an dem Vorderende (21) des Schutzkörpers (20) an den Vorderenden der zwei Fortsätze (35', 35") verbunden sind.

9. Vorrichtung nach einem oder mehreren der vorhergehenden Ansprüche, wobei in der Nähe des Hinterendes (22) des Schutzkörpers jeder Schlitz (24) einen ersten Abschnitt mit einem geweiteten Lumen (26) aufweist, der einen Blockiersitz definiert, wobei in den Sitz der entsprechende Flügel (12) des Nadelkörpers eingreift, wenn er in die geschützte Nadelposition gebracht wird, und wobei in der Nähe des Vorderendes (21) des Schutzkörpers (20) jeder Schlitz (24) einen zweiten Abschnitt mit einem geweiteten Lumen (26") aufweist, der einen Haltesitz (37) für den Nadelkörper definiert, wenn sich der letztere in einer blanken Nadelposition befindet.

10. Hohler Nachverwendungs-Schutzkörper (20) der Vorrichtung nach Anspruch 1.

## Revendications

1. Dispositif pour pénétrer dans un vaisseau sanguin en utilisant une aiguille à ailettes dotée d'une protection après utilisation, comprenant :
- un corps d'aiguille à ailettes (10) doté d'un élément de support tubulaire (11) à partir duquel deux ailettes (12) s'étendent latéralement en directions opposées, et depuis lequel la partie distale (14) d'une aiguille (13) s'étend longitudinalement pour pénétrer dans un vaisseau sanguin ;
- un corps de protection creux (20), qui s'étend dans la direction longitudinale entre une extrémité avant (21) et une extrémité arrière (22), définissant une chambre intérieure (23) à l'intérieur de laquelle le corps d'aiguille est inséré de façon à coulisser, avec les deux ailettes qui dépassent du corps de protection au travers de deux fentes de coulissement latérales (24) qui s'étendent à l'opposé l'une de l'autre entre lesdites deux extrémités (21, 22),
le corps d'aiguille (10) étant mobile de façon à coulisser le long de la chambre intérieure (23) à partir d'une position d'aiguille nue, dans laquelle il est positionné à proximité de l'extrémité avant (21) du corps de protection (20) avec la partie distale (14) de l'aiguille sortant par une ouverture de sortie (25) constituée dans celle-ci, jusqu'à une position d'aiguille protégée, dans laquelle il est positionné à proximité de l'extrémité arrière (22) du corps de protection avec l'aiguille complètement rétractée dans ledit corps de protection, dans une partie du corps de protection creux qui délimite la chambre intérieure (23), à proximité de l'ouverture de sortie (25), une ouverture supplémentaire (40) étant aménagée, laquelle ouverture supplémentaire s'étend dans la direction longitudinale vers l'extrémité avant (21) aussi loin que l'ouverture de sortie (25), les deux fentes (24) divisant le corps de protection (20) en deux demi-coques (20', 20"), ces dernières étant raccordées l'une à l'autre au moins à l'extrémité avant (21) dudit corps de protection de telle sorte que la chambre intérieure (23) du corps de protection creux (20) puisse être ouverte en écartant les deux demi-coques (20', 20") au niveau de la partie arrière (22) avec une rotation traversante autour d'un axe de rotation (X) passant à proximité de l'ouverture de sortie (25),
**caractérisé en ce que** ladite ouverture supplémentaire (40) est fermée au moins partiellement par une languette (30) qui s'étend de façon à se projeter depuis le corps de protection creux dans la direction longitudinale vers l'extrémité avant (21), ladite languette (30) étant mobile entre une position de repos dans laquelle la languette (30) ferme au moins partiellement l'ouverture supplémentaire (40) réduisant la lumière ouverte de ladite ouverture supplémentaire (40), et une position de fonctionnement dans laquelle la languette (30) est écartée du corps de protection creux de façon à laisser ouverte l'ouverture supplémentaire (40) et à élargir l'ouverture de sortie (25).

2. Dispositif selon la revendication 1, dans lequel dans la position de repos, la languette en projection (30) ferme ladite ouverture supplémentaire (40) tout au long de son extension longitudinale, l'extrémité libre (31) de ladite languette (30) concourant à définir le contour de l'ouverture de sortie (25).

3. Dispositif selon la revendication 1 ou 2, dans lequel la languette (30) est constituée d'un élément élastiquement flexible, approprié pour un déplacement élastique de ladite position de repos à ladite position de fonctionnement lorsqu'il est contraint dans cette direction et pour revenir automatiquement à la position de repos en l'absence de contraintes.

4. Dispositif selon la revendication 3, dans lequel la languette en projection (30) a, au niveau d'une base de raccordement (32) avec le corps de protection (20), une incision (33) qui définit une ligne de courbure préférentielle de la languette (30), de préférence ladite incision (33) étant sensiblement transversale à l'extension longitudinale du corps de protection creux (20) et de ladite languette (30).

5. Dispositif selon la revendication 4, dans lequel l'incision (33) est constituée de façon à faciliter la courbure de la languette (30) vers l'extérieur de la chambre intérieure (23).

6. Dispositif selon une ou plusieurs des revendications précédentes, dans lequel l'ouverture supplémentaire (40) est définie longitudinalement par deux appendices (35', 35") du corps de protection creux (20) entre lesquels la languette en projection (30) est positionnée lorsque qu'elle est dans la position de repos.

7. Dispositif selon une ou plusieurs des revendications précédentes, dans lequel dans la configuration de chambre ouverte, les deux demi-coques (20', 20") peuvent être agencées l'une par rapport à l'autre de telle sorte que la languette (30) dans la position de repos soit sensiblement sur le même plan ou parallèle à un plan sur lequel se trouve l'ouverture de sortie (25).

8. Dispositif selon une ou plusieurs des revendications précédentes, dans lequel l'ouverture de sortie (25) est constituée sur une première demi-coque (20') et l'ouverture supplémentaire (40) est constituée sur la seconde demi-coque (20"), les deux demi-coques (20', 20") étant raccordée l'une à l'autre au moins à l'extrémité avant (21) du corps de protection (20) aux extrémités avant desdits deux appendices (35', 35").

9. Dispositif selon une ou plusieurs des revendications précédentes, dans lequel à proximité de l'extrémité arrière (22) du corps de protection, chaque fente (24) a une première section dotée d'une lumière élargie (26) qui définit un siège de blocage à l'intérieur duquel l'ailette correspondante (12) du corps d'aiguille est en prise lorsqu'il est amené dans la position d'aiguille protégée et dans lequel à proximité de l'extrémité avant (21) du corps de protection (20), chaque fente (24) a une seconde section dotée d'une lumière élargie (26") qui définit un siège de retenue (37) pour le corps d'aiguille lorsque ce dernier est dans une position d'aiguille nue.

10. Corps de protection creux, après utilisation, (20) du dispositif selon la revendication 1.
